# EUROPEAN PATENT SPECIFICATION

(11) **EP 4 147 739 B1**
(45) Date of publication and mention of the grant of the patent: **03.09.2025**
(21) Application number: 21822758.5
(22) Date of filing: 26.03.2021
(51) Int. Cl.: A61M 25/00, A61M 25/01

(54) **CATHETER**
KATHETER
CATHÉTER

(30) Priority: 12.06.2020 JP 2020102078
(43) Date of publication of application: 15.03.2023
(73) Proprietor: TERUMO Kabushiki Kaisha, Tokyo 151-0072 (JP)
(72) Inventor: ITO, Takashi, Fujinomiya-shi, Shizuoka 418-0015 (JP); YAMAMOTO, Keiichiro, Elkton, Maryland 21921 (US)
(74) Representative: Casalonga
(86) International application number: PCT/JP2021/012980
(87) International publication number: WO 2021/250980

(56) References cited:
- JP-A- 2012 249 811
- JP-A- 2012 249 811
- JP-A- 2019 022 538
- JP-A- 2019 037 572
- JP-A- 2020 080 930
- US-A1- 2015 314 108
- US-A1- 2016 279 385
- US-A1- 2018 093 068

## Description

### Technical Field

The present invention relates to a catheter used in a lumen such as a blood vessel. In particular, the present invention relates to a catheter according to the preamble of claim 1, such as it is e.g. known from US2018/093068 A1, US2015/314108 A1 or JP2019 037572 A.

### Background Art

In recent years, for a reason that surgical invasiveness is fairly low, a treatment in a lumen such as a blood vessel using a catheter has been actively performed. For example, in general, the catheter used by being selectively introduced into a complexly branched blood vessel in a body is selectively pushed in along a guide wire introduced in advance into the blood vessel to circulate a therapeutic drug, a diagnostic contrast agent, or the like from a proximal side to a distal side.

An radiopaque marker is disposed in a shaft such that a position of the catheter in the lumen can be recognized from the outside of the body. For example, PTL 1 describes a catheter in which a marker is fixed to an outer peripheral surface of a shaft by punching. PTL 2 describes a catheter in which a marker is disposed on an inner peripheral surface of a shaft and from which a portion protruding in a radial direction on an outer surface of the shaft is removed such that the catheter is flat.

The shaft is formed thin such that the shaft can be inserted into the blood vessel or the like. Alternatively, in order to ensure pushing performance (pushability), torque transmission performance, or the like, a reinforcement body may be provided in which a wire is braided. Meanwhile, for example, when the shaft is inserted from a radial artery of a hand and treatment of a lower limb is performed, a long shaft is required. When the shaft is long, it is difficult to transmit a pushing force and a torque that are applied to a proximal portion of the shaft to a distal portion of the shaft. Therefore, in order to improve the pushing performance and the torque transmission performance, for example, it is conceivable to increase a wire diameter of the wire of the reinforcement body.

### Citation List

### Patent Literature

PTL 1: JP-A-2000-237319
PTL 2: JP-A-6-197974

### Summary of Invention

### Technical Problem

When the wire diameter of the braided wire is increased, a thickness of the shaft of the portion where the marker is provided is also increased when the marker is disposed in the shaft where the reinforcement body is embedded.

The invention has been made to solve the above-described problems, and an object of the invention is to provide a catheter capable of preventing an increase in a thickness of a shaft while disposing an radiopaque marker on the shaft provided with a reinforcement body.

### Solution to Problem

This problem is solved by a catheter according to independent claim 1. The dependent claims relate to advantageous embodiments.

### Advantageous Effect of Invention

In the catheter configured as described above, the outer diameter of the shaft provided with the reinforcement body and the radiopaque marker is not too large, and/or the inner diameter of the shaft is not too small. Therefore, it is possible to prevent an increase in the thickness of the shaft while disposing the marker on the shaft provided with the reinforcement body.

A thickness of an outer layer of the shaft in the portion where the marker is disposed may be the same as a thickness of an outer layer of the shaft in the portion adjacent to the marker in the axial direction of the shaft. Accordingly, it is possible to prevent an increase in the thickness of the shaft while making the thickness of the outer layer uniform regardless of the presence or absence of the marker.

The marker may include at least one convex portion on at least an inner surface side. Accordingly, the marker is firmly fixed to the shaft. Therefore, detachment of the marker from the shaft can be prevented.

An effective length of the shaft may be 2100 mm or more. Accordingly, the catheter can easily reach an artery of a lower limb from an artery of an arm.

### Brief Description of Drawings

[Fig. 1] Fig. 1 is a plan view showing a catheter.
[Fig. 2] Fig. 2 is a cross-sectional view showing a distal portion of the catheter.
[Fig. 3] (A) and (B) of Fig. 3 are cross-sectional views showing the distal portion of a catheter according to an illustrative example not falling under the scope of the claims, (A) of Fig. 3 is a cross-sectional view taken along a line A-A in Fig. 2, and (B) of Fig. 3 is a cross-sectional view taken along a line B-B in Fig. 2.
[Fig. 4] Fig. 4 is a plan view showing the catheter of Fig. 3 through an outer layer of the catheter.
[Fig. 5] Fig. 5 is a plan view showing an embodiment of the catheter according to the invention.
[Fig. 6] Fig. 6 is a plan view showing another modification of the catheter not falling under the scope of the claims.

### Description of Embodiments

Hereinafter, the invention will be described with reference to the drawings. For convenience of explanation, dimensions in the drawings may be exaggerated and may be different from actual dimensions. In the specification and the drawings, components having substantially the same functional configuration are designated by the same reference signs, and a duplicate description thereof will be omitted. In the present specification, a side where a catheter is inserted into a biological lumen is referred to as a "distal side", and a side to be operated is referred to as a "proximal side".

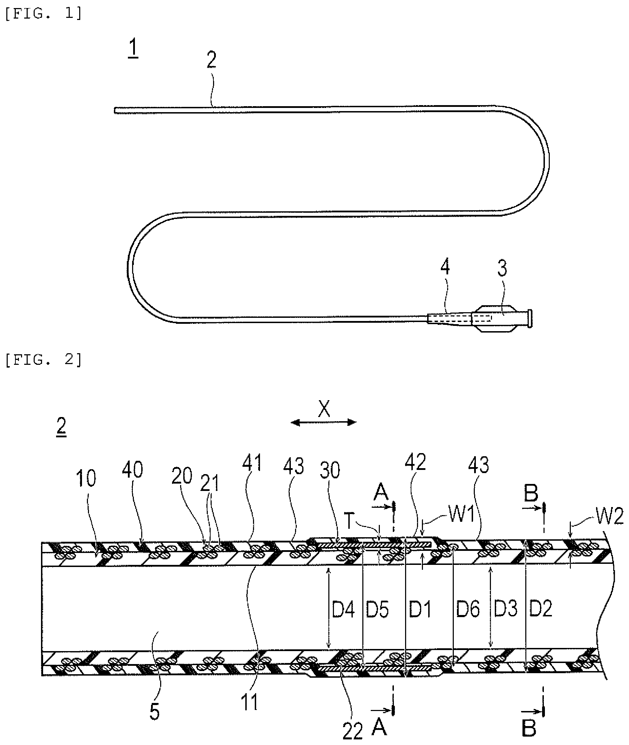

A catheter 1 is introduced into a blood vessel from a radial artery of an arm, inserted into an artery of a lower limb, and used for treatment, diagnosis, and the like. The artery of the lower limb is an artery near and more peripheral to an aortoiliac bifurcated portion. As shown in Fig. 1, a catheter 1 includes a long shaft 2, a hub 3 coupled to a proximal end of the shaft 2, and an strain relief 4 provided at a connection portion between the shaft 2 and the hub 3.

As shown in Figs. 1 to 4, the shaft 2 is a flexible tubular member, and a lumen 5 is formed inside from the proximal end to a distal end. A guide wire is inserted into the lumen 5 when the catheter 1 is inserted into a blood vessel. The lumen 5 can also be used as a passage for a drug solution, an embolic substance, a contrast agent, a medical instrument, or the like.

An effective length of the shaft 2 is not particularly limited, but is preferably 1500 mm to 2600 mm, more preferably 1800 mm to 2300 mm, still more preferably 2100 mm to 2300 mm, and is 2100 mm. Accordingly, the catheter 1 can reach the artery of the lower limb from the artery of the arm. The effective length of the shaft 2 is a length of a portion that can be inserted into a blood vessel, a sheath, or the like. The effective length is a length from a distal-most end of the strain relief 4 to a distal-most end of the shaft 2. The effective length of the shaft 2 is preferably 650 mm or more when the catheter is introduced from a femoral artery, and is preferably 300 mm or more when the catheter approaches from a distal portion of a dorsalis pedis artery or a posterior tibial artery.

The shaft 2 is comprised by a plurality of layers, and includes an inner layer 10 forming an inner surface 11 of the lumen 5, a reinforcement body 20 formed on an outer side of the inner layer 10, a marker 30 disposed on an outer side of the reinforcement body 20 in a radial direction, and an outer layer 40 formed on an outer side of the inner layer 10 and the reinforcement body 20. A thickness of the shaft 2 is larger than a sum of a thickness of the reinforcement body 20 and a thickness of the marker 30. Therefore, the reinforcement body 20 and the marker 30 can be completely embedded inside the shaft 2.

An inner diameter of the inner layer 10 is not particularly limited, but is preferably 0.4 mm to 1.2 mm, more preferably 0.5 mm to 1.2 mm, still more preferably 0.95 mm to 1.2 mm, and is 1.05 mm in the present embodiment. The inner diameter of the inner layer 10 may be smaller than a portion adjacent in an axial direction X of the shaft 2 at a position covered by the marker 30.

The lumen 5 is formed inside the inner layer 10. As a constituent material of the inner layer 10, a thermoplastic resin, a thermosetting resin, or the like can be used, and a fluorine-based resin such as polytetrafluoroethylene (PTFE), a low-friction material such as high-density polyethylene (HDPE), or the like is preferable.

The reinforcement body 20 is formed by braiding a plurality of wires 21 in a tubular shape with a gap around an outer periphery of the inner layer 10. In the reinforcement body 20, a small diameter portion 22 having a locally small outer diameter is formed in order to dispose the marker 30. A length of the small diameter portion 22 in the axial direction X is preferably larger than or equal to a length of the marker 30 in the axial direction X such that the marker 30 can be accommodated. The reinforcement body 20 may be formed by winding the wires 21 in horizontal winding in the same direction, or winding the wires 21 while changing a winding direction, such as right-handed winding and left-handed winding. A winding pitch, an interstitial distance, an inclination angle with respect to a peripheral direction, and the like may be changed depending on a position. The configuration is not particularly limited. The small diameter portion 22 may not be formed.

A difference (D5-D6) between a maximum outer diameter D5 of the marker 30 and a maximum outer diameter D6 of the reinforcement body 20 in the portion adjacent to the marker 30 in the axial direction X of the shaft 2 is smaller than twice a thickness T of the marker 30.

A wire diameter (diameter) of the wire 21 of the reinforcement body 20 is not particularly limited, but is preferably 0.03 mm to 0.08 mm, more preferably 0.03 mm to 0.06 mm, still more preferably 0.04 mm to 0.06 mm, and is 0.05 mm in the present embodiment.

As the wire 21 used for the reinforcement body 20, a metal wire made of metal such as stainless steel, platinum (Pt) and tungsten (W), resin fiber, carbon fiber, glass fiber, or the like can be used, or the plurality of these wires 21 may be used in combination.

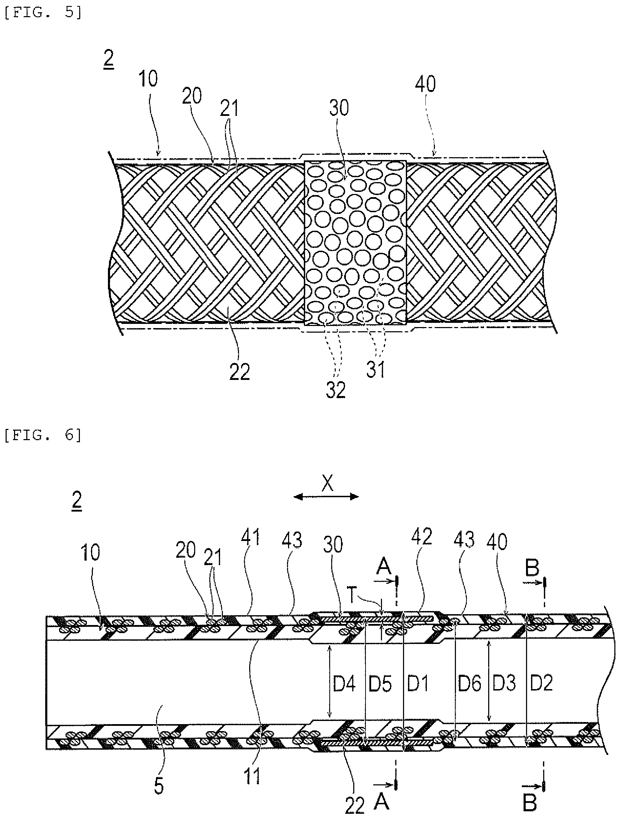

The marker 30 is an radiopaque (X-ray contrast) tube body that enters the small diameter portion 22 from the outer side in the radial direction. When the marker 30 is disposed, after the reinforcement body 20 is disposed around the outer periphery of the inner layer 10, a tube body formed of a material containing an radiopaque substance is disposed in a manner of surrounding the inner layer 10 and the reinforcement body 20. Thereafter, the tube body is crimped from the outer side in the radial direction to form a plurality of concave portions 31 and a plurality of convex portions 32 on an inner peripheral surface of the tube body, and the tube body is reduced in diameter to dispose the marker 30 in the shaft 2. Therefore, a cross-sectional shape of the marker 30 in a cross section orthogonal to the axial direction X of the shaft 2 is non-circular. In the present embodiment, the concave portion 31 extends in the axial direction X of the shaft 2 and is formed in a groove shape. The convex portion 32 extends in the axial direction X of the shaft 2 and is formed in a beam shape. The concave portion 31 and the convex portion 32 are arranged substantially uniformly in the peripheral direction of the marker 30. The number of the concave portions 31 and the number of the convex portions 32 are not particularly limited, but a cross-sectional shape of the marker 30 approaches a circular shape as the number increases. The concave portion 31 may not be in the groove shape. The convex portion 32 may not be in the beam shape. For example, in the embodiment according to the invention shown in Fig. 5, the concave portions 31 and the convex portions 32 are randomly formed.

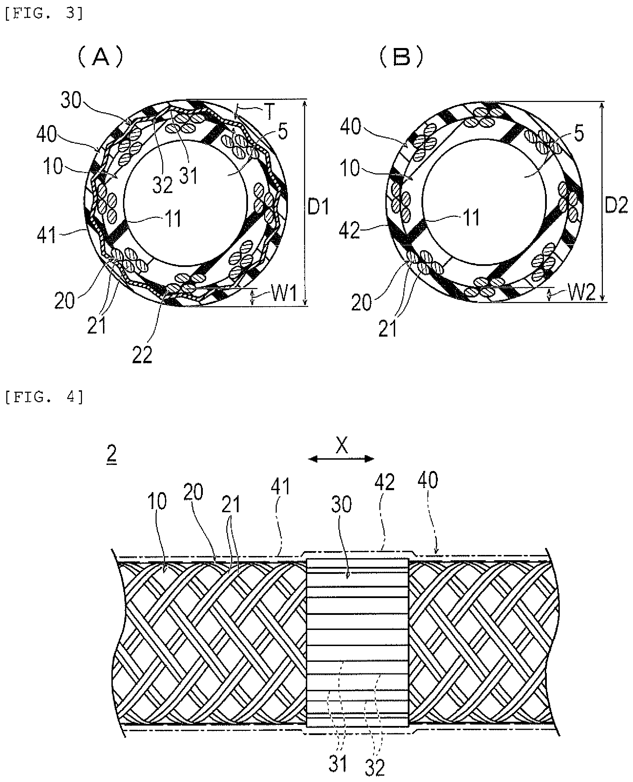

As shown in Fig. 2 and (A) and (B) of Fig. 3, the thickness T of the marker 30 can be an average value of the thicknesses T of the marker 30 in the cross section orthogonal to an axis of the shaft 2 at a predetermined position (for example, a center or an end portion in the axial direction X). The average value of the thicknesses T of the marker 30 in the cross section orthogonal to the axis of the shaft 2 may be, for example, a value obtained by dividing a cross-sectional area of the marker 30 in the cross section by a length (for example, an average value of a peripheral length of the inner peripheral surface of the marker 30 and a peripheral length of the outer peripheral surface of the marker 30) of the marker 30 in the peripheral direction.

The thickness T of the marker 30 is not particularly limited, but is preferably 0.02 mm to 0.1 mm, more preferably 0.03 mm to 0.08 mm, still more preferably 0.03 mm to 0.04 mm, and is 0.03 mm in the present embodiment.

As a constituent material of the marker 30, a material kneaded with an X-ray contrast agent such as platinum, gold, silver, tungsten, iridium, or metal powders of an alloy thereof, or barium sulfate, bismuth oxide, or a coupling compound thereof can be suitably used.

As shown in Figs. 2 to 4, the outer layer 40 is a tubular member that covers the outer periphery of the inner layer 10, the reinforcement body 20, and the marker 30. The outer layer 40 forms an outer surface 41 that is a surface on the outer side of the shaft 2 in the radial direction. The outer surface 41 includes an outer layer convex portion 42 formed at a position where the marker 30 is covered. An outer diameter D1 of the outer layer convex portion 42 is larger than an outer diameter D2 of the portion adjacent to the outer layer convex portion 42 in the axial direction X of the shaft 2. An outer diameter difference (D1-D2), which is a difference between the outer diameter D1 of the outer layer convex portion 42 and the outer diameter D2 of the portion (adjacent portion 43) adjacent to the outer layer convex portion 42 in the axial direction X, is smaller than twice the thickness T of the marker 30.

The outer diameter D1 of the outer layer convex portion 42 is not particularly limited, but is preferably 0.7 mm to 1.6 mm, more preferably 1.3 mm to 1.5 mm, still more preferably 1.36 mm to 1.46 mm, and is 1.41 mm in the present embodiment.

The outer diameter D2 of the portion adjacent to the outer layer convex portion 42 is not particularly limited, but is preferably 0.7 mm to 1.6 mm, more preferably 1.3 mm to 1.5 mm, still more preferably 1.35 mm to 1.45 mm, and is 1.40 mm in the present embodiment. The outer diameter difference (D1-D2), which is the difference between the outer diameter D1 and the outer diameter D2, is preferably 0 mm to 0.06 mm, more preferably 0 mm to 0.03 mm, still more preferably 0 mm to 0.01 mm, and is 0.01 mm in the present embodiment.

As a constituent material of the outer layer 40, for example, a thermoplastic resin such as a polymer material such as polyolefin (for example, polyethylene, polypropylene, polybutene, an ethylene-propylene copolymer, an ethylene-vinyl acetate copolymer, an ionomer, or a mixture of two or more thereof), polyvinyl chloride, polyamide, a polyester elastomer, a polyamide elastomer, polyurethane, a polyurethane elastomer, polyimide, or fluororesin, or a mixture thereof, and a thermosetting resin such as epoxy resin can be used. The radiopaque substance may be mixed in the outer layer 40.

The proximal portion of the shaft 2 is liquid-tightly fixed to the hub 3 by an adhesive, heat-welding, a fastener (not shown) or the like. The hub 3 functions as an insertion port of a guide wire or a medical instrument into the lumen 5, an injection port of a drug solution, an embolic substance, a contrast agent, or the like into the lumen 5, or the like, and also functions as a grip portion when the catheter 1 is operated. A constituent material of the hub 3 is not particularly limited, and for example, a thermoplastic resin such as polycarbonate, polyamide, polysulfone, polyarylate, or a methacrylate-butylene-styrene copolymer can be suitably used.

The strain relief 4 is made of an elastic material that surrounds the periphery of the shaft 2, and prevents kinking of the shaft 2 at the connection portion between the shaft 2 and the hub 3. As a constituent material of the strain relief 4, for example, natural rubber, silicone resin, or the like can be suitably used.

Next, a method (not claimed) for manufacturing the catheter 1 according to the present embodiment will be described.

First, a long core wire having an outer diameter equal to the inner diameter of the inner layer 10 is prepared. Next, the inner layer 10 is formed on the core wire. The inner layer 10 may not be formed by extrusion molding, or may be formed by dip molding. Alternatively, the core wire may be inserted into the inner layer 10, which is the tube body.

Thereafter, the reinforcement body 20 is formed in a manner of covering at least a part of the inner layer 10. The reinforcement body 20 is formed by continuously winding the plurality of wires 21 on the inner layer 10 using a braid machine (braider).

Next, the marker 30 is disposed on the outer side of the inner layer 10 and the reinforcement body 20, and the marker 30 is crimped by a crimping machine to bite into the reinforcement body 20. Accordingly, the reinforcement body 20 receives a force from the outer side in the radial direction and is reduced in diameter, and the small diameter portion 22 is formed. The small diameter portion 22 may not be formed in the marker 30. Thereafter, the outer layer 40 including the outer layer convex portion 42 is formed on the outer side of the inner layer 10, the reinforcement body 20, and the marker 30. Accordingly, the outer diameter difference (D1-D2), which is the difference between the outer diameter D1 of the shaft 2 in the portion where the marker 30 is disposed and the outer diameter D2 of the shaft 2 in the portion adjacent to the marker 30 in the axial direction X of the shaft 2, is smaller than twice the thickness T of the marker 30. In the present embodiment, the portion adjacent to the marker 30 in the axial direction X is a portion on the distal side and/or the proximal side of the marker 30. An inner diameter difference (D3-D4), which is a difference between an inner diameter D3 of the shaft 2 in the portion adjacent to the marker 30 in the axial direction X of the shaft 2 and an inner diameter D4 of the shaft 2 in the portion where the marker 30 is disposed, is smaller than twice the thickness T of the marker 30. The inner diameter D3 and the inner diameter D4 may be equal or may not be equal to each other. A method for forming the outer layer 40 is not particularly limited. For example, the outer layer 40 may be formed by the extrusion molding, or may be formed by the dip molding. Alternatively, the outer layer 40 may be formed by disposing the tube body as a material of the outer layer 40 on the outer side of the inner layer 10, the reinforcement body 20, and the marker 30, and then heating the tube body by covering the tube body with a heat-shrinkable tube. The tube body is softened or melted by heating, and is closely bonded to the outer side of the inner layer 10, the reinforcement body 20, and the marker 30 by a contraction force of the heat-shrinkable tube. Thereafter, the heat-shrinkable tube subjected to heat shrinkage is removed.

After the outer layer 40 is formed, the core wire is pulled out from the lumen 5 of the inner layer 10. Thereafter, the hub 3 and the strain relief 4 are attached to the shaft 2, and other members (for example, distal chips) are attached as necessary to complete the catheter 1.

As described above, the catheter 1 according to the present embodiment is the catheter 1 that includes the shaft 2 including the lumen 5 communicating from the distal end to the proximal end. The shaft 2 includes the reinforcement body 20 including the plurality of wires 21 that are disposed in at least a part between the inner surface 11 of the shaft 2 forming the lumen 5 and the outer surface 41 of the shaft 2 and that are braided in the tubular shape, and at least one or more radiopaque markers 30 that are disposed between the reinforcement body 20 and the outer surface 41. The outer diameter difference (D1-D2), which is the difference between the outer diameter D1 of the shaft 2 in the portion where the marker 30 is disposed and the outer diameter D2 of the shaft 2 in the portion adjacent to the marker 30 in the axial direction X of the shaft 2, is smaller than twice the thickness T of the marker 30. Accordingly, the outer diameter of the shaft 2 provided with the reinforcement body 20 and the radiopaque marker 30 is not too large. Therefore, it is possible to prevent an increase in the thickness of the shaft 2 while disposing the marker 30 in the shaft 2 provided with the reinforcement body 20. By preventing the increase in the thickness of the shaft 2, the inner diameter of the shaft 2 can be made as large as possible while the outer diameter of the shaft 2 can be made as small as possible. Such a catheter 1 is particularly effective when a blood vessel (for example, the radial artery) to be introduced is thin and a blood vessel (for example, the artery of the lower limb) to be treated or diagnosed is thick.

The difference (D5-D6) between the maximum outer diameter D5 of the marker 30 and the maximum outer diameter D6 of the reinforcement body 20 in the portion adjacent to the marker 30 in the axial direction of the shaft 2 is smaller than twice the thickness T of the marker 30. Accordingly, the outer diameter of the shaft 3 provided with the reinforcement body 20 and the radiopaque marker 30 is not too large, and the inner diameter of the shaft 2 is not too small. Therefore, it is possible to prevent an increase in the thickness of the shaft 2 while disposing the marker 30 in the shaft 2 provided with the reinforcement body 20.

A thickness W1 of the outer layer 40 of the shaft 2 in the portion where the marker 30 is disposed and a thickness W2 of the outer layer 40 of the shaft 2 in the portion adjacent to the marker 30 in the axial direction X of the shaft 2 may be the same. Accordingly, it is possible to prevent an increase in the thickness of the shaft 2 while making the thicknesses W1 and W2 of the outer layer 40 uniform regardless of the presence or absence of the marker 30. If the thicknesses W1 and W2 of the outer layer 40 are sufficient, the outer layer convex portion 42 may not be formed depending on the presence or absence of the marker 30. Therefore, although the thicknesses W1 and W2 of the outer layer 40 are thin and uniform, the outer diameter difference (D1-D2) may be smaller than the thickness of the marker 30.

The marker 30 includes at least one convex portion 32 on at least an inner surface side. Accordingly, the marker 30 is firmly fixed to the shaft 2. Therefore, detachment of the marker 30 from the shaft 2 can be prevented.

The effective length of the shaft 2 is 2100 mm or more. Accordingly, the catheter 1 can easily reach the artery of the lower limb from the artery of the arm.

The invention is not limited to the embodiment described above, and various modifications can be made by those skilled in the art within a scope of the technical idea of the invention. For example, in the embodiment described above, the number of markers 30 disposed in the shaft 2 is one, but may be two or more. The catheter 1 may be inserted from a blood vessel other than the artery of the arm. The catheter 1 may be used for the treatment or the diagnosis of the blood vessel other than the artery of the lower limb. The catheter 1 may be inserted into a bile duct, trachea, esophagus, urethra, or other biological lumens or a lumen in a living body and used for the treatment, the diagnosis, or the like.

As in another modification (not falling under the scope of the claims) shown in Fig. 6, the inner diameter D4 of the shaft 2 in the portion where the marker 30 is disposed may be smaller than the inner diameter D3 of the adjacent portion 43 adjacent to the marker 30 in the axial direction X of the shaft 2. The inner diameter difference (D3-D4), which is the difference between the inner diameter D3 and the inner diameter D4, is preferably smaller than twice the thickness T of the marker 30. Accordingly, the inner diameter of the shaft 2 provided with the reinforcement body 20 and the radiopaque marker 30 is not too small. Therefore, it is possible to prevent an increase in the thickness of the shaft 2 while disposing the marker 30 in the shaft 2 provided with the reinforcement body 20. By preventing an increase in the thickness of the shaft 2, the inner diameter of the shaft 2 can be made as large as possible while the outer diameter of the shaft 2 can be made as small as possible. Such a catheter 1 is particularly effective when a blood vessel (for example, the radial artery) to be introduced is thin and a blood vessel (for example, the artery of the lower limb) to be treated or diagnosed is thick.

One of the outer diameter difference (D1-D2) and the inner diameter difference (D3-D4) alone may be smaller than twice the thickness T of the marker 30. Alternatively, both the outer diameter difference (D1-D2) and the inner diameter difference (D3-D4) may be smaller than twice the thickness T of the marker 30.

All of the difference (D5-D6) between the maximum outer diameter D5 of the marker 30 and the maximum outer diameter D6 of the reinforcement body 20 in the portion adjacent to the marker 30 in the axial direction of the shaft 2, the outer diameter difference (D1-D2) and the inner diameter difference (D3-D4) may be smaller than twice the thickness T of the marker 30.

### Reference Signs List

1 catheter
2 shaft
3 hub
4 strain relief
5 lumen
10 inner layer
11 inner surface
20 reinforcement body
21 wire
22 small diameter portion
30 marker
31 concave portion
32 convex portion
40 outer layer
41 outer surface
42 outer layer convex portion
43 adjacent portion
D1 outer diameter of outer layer convex portion
D2 outer diameter in portion adjacent to outer layer convex portion
D3 inner diameter of shaft in portion adjacent to marker
D4 inner diameter of shaft in portion where marker is disposed
D5 maximum outer diameter of marker
D6 outer diameter of portion adjacent to marker
T thickness of marker
X axis direction

## Claims

1. A catheter (1) comprising:
a shaft (2) including a lumen (5) communicating from a distal end to a proximal end, wherein
the shaft (2) includes
a reinforcement body (20) including a plurality of wires (21) that are disposed on at least a part between an inner surface (11) of the shaft (2) forming the lumen (5) and an outer surface (41) of the shaft (2) and that are braided in a tubular shape, and
at least one or more radiopaque markers (30) that are disposed between the reinforcement body (20) and the outer surface (41),
a difference (D1 - D2) between an outer diameter (D1) of the shaft (2) in a portion where the marker (30) is disposed and an outer diameter (D2) of the shaft (2) in a portion adjacent to the marker (30) in an axial direction of the shaft (2) is defined as an outer diameter difference, and
a difference (D3 - D4) between an inner (D3) diameter of the shaft (2) in the portion adjacent to the marker (30) in the axial direction of the shaft (2) and an inner diameter (D4) of the shaft (2) in the portion where the marker (30) is disposed is defined as an inner diameter difference ( D3 - D4), and
at least one of the outer diameter difference and the inner diameter difference is smaller than twice a thickness (T) of the marker (30), i.e. (D1 - D2) < 2T and/or (D3 - D4) < 2T, and/or
a difference (D5 - D6) between a maximum outer diameter (D5) of the marker (30) and a maximum outer diameter (D6) of the reinforcement body (20) in a portion adjacent to the marker (30) in an axial direction of the shaft (2) is smaller than twice a thickness (T) of the marker (30), i.e. (D5 - D6) < 2 T,
**characterized in that**
a plurality of concave portions (31) and a plurality of convex portions (32) are randomly formed on the marker (30).

2. The catheter (1) according to claim 1, wherein
a thickness (W1) of an outer layer (40) of the shaft (2) in the portion where the marker (30) is disposed is the same as a thickness (W2) of an outer layer (40) of the shaft (2) in the portion adjacent to the marker (30) in the axial direction of the shaft (2), i.e. W1 = W2.

3. The catheter (1) according to any one of claims 1 to 2, wherein
an effective length of the shaft (2) is 2100 mm or more.

## Patentansprüche

1. Katheter (1), umfassend:
einen Schaft (2), der ein Lumen (5) umfasst, das von einem distalen Ende zu einem proximalen Ende in Verbindung steht, wobei
der Schaft (2) umfasst
einen Verstärkungskörper (20), der eine Vielzahl von Drähten (21) aufweist, die auf mindestens einem Teil zwischen einer Innenfläche (11) des Schaftes (2), die das Lumen (5) bildet, und einer Außenfläche (41) des Schaftes (2) angeordnet sind, und die zu einer röhrenförmigen Struktur geflochten sind, und
mindestens eine oder mehrere röntgendichte Markierungen (30), die zwischen dem Verstärkungskörper (20) und der Außenfläche (41) angeordnet sind,
wobei ein Unterschied (D1 - D2) zwischen einem Außendurchmesser (D1) des Schaftes (2) in einem Abschnitt, in dem die Markierung (30) angeordnet ist, und einem Außendurchmesser (D2) des Schaftes (2) in einem Abschnitt, der in einer axialen Richtung des Schaftes (2) an die Markierung (30) angrenzt, als ein Außendurchmesserunterschied definiert ist, und
ein Unterschied (D3 - D4) zwischen einem Innendurchmesser (D3) des Schaftes (2) in dem Abschnitt, der in der axialen Richtung des Schaftes (2) an die Markierung (30) angrenzt, und einem Innendurchmesser (D4) des Schaftes (2) in dem Abschnitt, in dem die Markierung (30) angeordnet ist, als ein Innendurchmesserunterschied (D3 - D4) definiert ist, und
mindestens einer von dem Außendurchmesserunterschied und dem Innendurchmesserunterschied kleiner als das Doppelte einer Dicke (T) der Markierung (30) ist, d. h., (D1 - D2) < 2T und/oder (D3 - D4) < 2T, und/oder
ein Unterschied (D5 - D6) zwischen einem maximalen Außendurchmesser (D5) der Markierung (30) und einem maximalen Außendurchmesser (D6) des Verstärkungskörpers (20) in einem Abschnitt, welcher in einer axialen Richtung des Schaftes (2) an die Markierung (30) angrenzt, kleiner als das Doppelte der Dicke (T) von der Markierung (30) ist, d. h., (D5 - D6) < 2 T,
**dadurch gekennzeichnet, dass**
eine Vielzahl von konkaven Abschnitten (31) und eine Vielzahl von konvexen Abschnitten (32) zufällig auf der Markierung (30) ausgebildet sind.

2. Katheter (1) nach Anspruch 1, wobei
eine Dicke (W1) einer Außenschicht (40) des Schaftes (2) in dem Abschnitt, in welchem die Markierung (30) angeordnet ist, gleich einer Dicke (W2) einer Außenschicht (40) des Schaftes (2) in dem Abschnitt ist, der in der axialen Richtung des Schaftes (2) an die Markierung (30) angrenzt, d. h. W1 = W2.

3. Katheter (1) nach einem der Ansprüche 1 bis 2, wobei
eine effektive Länge des Schaftes (2) 2.100 mm oder mehr beträgt.

## Revendications

1. Cathéter (1) comprenant :
une tige (2) comportant une lumière (5) communiquant d'une extrémité distale à une extrémité proximale, dans lequel la tige (2) comporte
un corps de renfort (20) comportant une pluralité de fils (21) qui sont disposés sur au moins une partie entre une surface intérieure (11) de la tige (2) formant la lumière (5) et une surface extérieure (41) de la tige (2) et qui sont tressés en forme tubulaire, et
au moins un ou plusieurs marqueurs radio-opaques (30) qui sont disposés entre le corps de renfort (20) et la surface extérieure (41),
une différence (D1 - D2) entre un diamètre extérieur (D1) de la tige (2) dans une partie où le marqueur (30) est disposé et un diamètre extérieur (D2) de la tige (2) dans une partie adjacente au marqueur (30) dans une direction axiale de la tige (2) est définie comme une différence de diamètre extérieur, et
une différence (D3 - D4) entre un diamètre intérieur (D3) de la tige (2) dans la partie adjacente au marqueur (30) dans la direction axiale de la tige (2) et un diamètre intérieur (D4) de la tige (2) dans la partie où le marqueur (30) est disposé est définie comme une différence de diamètre intérieur (D3 - D4), et
au moins l'une parmi la différence de diamètre extérieur et la différence de diamètre intérieur est inférieure à deux fois une épaisseur (T) du marqueur (30), à savoir (D1 - D2) < 2T et/ou (D3 - D4) < 2T, et/ou
une différence (D5 - D6) entre un diamètre extérieur maximal (D5) du marqueur (30) et un diamètre extérieur maximal (D6) du corps de renfort (20) dans une partie adjacente au marqueur (30) dans une direction axiale de la tige (2) est inférieure à deux fois une épaisseur (T) du marqueur (30), à savoir (D5 - D6) < 2 T,
**caractérisé en ce que**
une pluralité de parties concaves (31) et une pluralité de parties convexes (32) sont formées aléatoirement sur le marqueur (30).

2. Cathéter (1) selon la revendication 1, dans lequel
une épaisseur (W1) d'une couche extérieure (40) de la tige (2) dans la partie où le marqueur (30) est disposé est la même qu'une épaisseur (W2) d'une couche extérieure (40) de la tige (2) dans la partie adjacente au marqueur (30) dans la direction axiale de la tige (2), c'est-à-dire W1 = W2.

3. Cathéter (1) selon l'une quelconque des revendications 1 à 2, dans lequel
une longueur effective de la tige (2) est de 2100 mm ou plus.
